Europäisches Patentamt

**European Patent Office**

**Office européen des brevets**

(11) Numéro de publication: **0 283 675 B1**

(12) **FASCICULE DE BREVET EUROPEEN**

(45) Date de publication de fascicule du brevet: **10.03.93**    (51) Int. Cl.5: **A61K 7/16**

(21) Numéro de dépôt: **88101292.6**

(22) Date de dépôt: **29.01.88**

(54) **Utilisation de glycopeptides lactique en hygiene bucco-dentaire.**

(30) Priorité: **26.02.87 CH 732/87**

(43) Date de publication de la demande:
**28.09.88 Bulletin  88/39**

(45) Mention de la délivrance du brevet:
**10.03.93 Bulletin  93/10**

(84) Etats contractants désignés:
**AT BE DE ES FR GB IT LU NL SE**

(56) Documents cités:
**FR-A- 1 580 913**
**FR-A- 2 425 809**
**US-A- 2 154 168**

**CHEMICAL ABSTRACTS, vol. 99, no. 24, décembre 1983, page 327, résumé no. 200364c, Columbus, Ohio, US; & JP-A-58 148 811 (KAO CORP.) 05-09-1983**

**CHEMICAL ABSTRACTS, vol. 101, 1984, page 509, résumé no. 22354z, Columbus, Ohio, US; S.I. ALEINIK et al.: "Glycopeptide from K-casein and its effect on protein utilization", & VOPR. PITAN. 1984, (2), 47-50**

(73) Titulaire: **SOCIETE DES PRODUITS NESTLE S.A.**
**Case postale 353**
**CH-1800 Vevey(CH)**

(72) Inventeur: **Neeser, Jean-Richard**
**Rue du Temple 34**
**CH-1010 Lausanne(CH)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention se rapporte à l'inhibition de l'adhésion des bactéries de la plaque et de la carie dentaires.

Actinomyces viscosus et Streptococcus sanguis sont des bactéries peuplant la cavité buccale responsables de l'initiation et de la formation de la plaque dentaire. Avec Actinomyces naeslundii, elles forment un groupe de bactéries capables de coloniser les premières la cavité buccale. Elles jouent un rôle capital dans la cascade d'événements conduisant aux maladies associées à la plaque dentaire comme les caries de racine, les gingivites, les périodontites et la paradontolyse. Par ailleurs, Streptococcus mutans est le pathogène principal associé à la carie dentaire qui se développe une fois la plaque dentaire initiée.

Parmi les facteurs entrant dans les mécanismes de formation de la plaque dentaire par les souches A. viscosus et S. sanguis, il faut citer leurs propriétés d'adhérer aux cellules épithéliales buccales, à la surface des dents recouvertes de salive, ainsi que leur tendance à former entre elles des co-aggrégats, ainsi qu'avec A. naeslundii. Bien que ces mécanismes soient très complexes, il a été observé que A. naeslundii reconnaissait un récepteur contenant du galactose ou de la galactosamine sur les cellules épithéliales grâce à des adhésines également exprimées par A. viscosus et appelées pili de type 2. D'autres travaux ont montré que les réactions de co-aggrégation entre S. sanguis et ces deux espèces d'Actinomyces pouvaient également être basées sur une reconnaissance de type pili de type 2 / galactose.

En simplifiant, d'une part il apparaît dans l'état de la technique que l'inhibiteur classique de l'adhésion bactérienne régie par les pili de type 2 est le lactose par l'intermédiaire de sa partie galactose. D'autre part, des mécanismes d'adhésion basés sur d'autres facteurs faisant par exemple intervenir la surface solide des dents, entrent en jeu dans la formation de la plaque dentaire.

Le brevet des Etats-Unis No. 4.579.736 se rapporte à la préparation d'un facteur inhibiteur de la croissance et du métabolisme des bactéries formant la plaque dentaire s. sanguis et A. viscosus, consistant à briser les parois cellulaires de Actinobacillus actinomycetemcomitans (qui est une bactérie présente dans la phase finale des périodontites) par un traitement aux ultrasons et à recueillir le facteur en question par centrifugation puis décantation de la phase liquide surnageante, purification de celle-ci par dialyse et séchage par lyophilisation.

La demande de brevet européen No. 184.121 concerne un additif ménageant les dents destiné à être incorpore dans les aliments et les médicaments contenant du saccharose, lequel additif consiste essentiellement en un mélange de lactose et de galactose obtenu par fermentation du petit-lait à l'aide de Lactobacillus leichmannii.

Dans CA, vol. 101, 1984, p.509, résumé No 22354z, on décrit l'application des k-caséinoglycopeptides en tant qu'agent antisécrétoires en diététique et en nutrition consistant à créer les conditions favorisant l'utilisation des protéines au premier âge par administration intraveineuse ou intraintestinale.

Nous avons trouvé que des glycopeptides d'origine lactique possèdent une activité inhibitrice remarquable des hémagglutinations régies par les pili de type 2 d'une part, et de l'adhésion de A.viscosus, S.sanguis et S.mutans sur surface plastique d'autre part, activité bien supérieure à celles observées pour le lactose ou le galactose.

L'invention concerne l'utilisation des kappa-caséinoglycopeptides ou de leurs dérivés désialylés en tant qu'agents anti-plaque dentaire ainsi que de leurs dérivés désialylés en tant qu'agents anti-carie dentaire.

L'invention concerne également l'utilisation des kappacaséinoglycopeptides en combinaison avec un support adapté à l'administration par voie orale ou topique pour l'obtention d'une composition destinée à traiter la carie dentaire.

Selon l'invention, on entend par kappa-caséino-glycopeptides aussi bien un caséino-macroglycopeptide qui est le composant hydrosoluble provenant de l'hydrolyse de la kappa-caséine par la présure, qu'un caséino-glycopeptide obtenu par la protéolyse d'un tel caséino-macroglycopeptide.

Par dérivés désialylés, on entend les dérivés obtenus à partir des glycopeptides précédents par élimination plus ou moins poussée des acides sialiques, soit les acides N-acétylneuraminiques et N-glycollylneuraminiques des chaînes oligosaccharidiques.

Un groupe préféré de principes actifs est constitué par les kappa-caséinomacropeptides et leurs dérivés désialylés dont l'activité est supérieure à celle des composés correspondants ayant subi une protéolyse.

Un tel principe actif peut être préparé en faisant réagir une matière première d'origine lactique ayant été préalablement hydrolysée par la présure, avec l'acide trichloracétique de manière à former un précipité et un surnageant, en séparant le surnageant, en le soumettant à une dialyse, en purifiant le cas échéant ledit surnageant dialysé par filtration sur gel et en le séchant.

Comme matière première d'origine lactique, on peut citer, à titre indicatif:

2

- le produit de l'hydrolyse par la présure d'une caséine native obtenue par précipitation acide du lait écrémé par un acide minéral ou des ferments acidifiants, le cas échéant avec addition d'ions calcium,
- le produit de l'hydrolyse par la présure d'un caséinate,
- un petit-lait doux (obtenu après séparation de la caséine coagulée par la présure),
- un petit-lait doux déminéralisé, par exemple par électrodialyse et/ou échange d'ions et/ou osmose inverse plus ou moins délactosé,
- un concentré de protéines de petit-lait obtenu par ultrafiltration, puis diafiltration (ultrafiltration avec lavage) de petit-lait doux, cette dernière matière première étant préférée.

On peut mettre en oeuvre les produits laitiers précédents obtenus à partir du lait de toutes femelles laitières, de préférence de vache, de chèvre ou de brebis.

Les dérivés désialylés peuvent être préparés en faisant subir au caséino-macroglycopeptide obtenu une étape ultérieure de désialylation dans laquelle on débarasse la partie oligosaccharidique du macro-glycopeptide des acides sialiques. Pour ce faire, on peut traiter le caséino-macroglycopeptide brut, c'est-à-dire non préalablement purifié par filtration sur gel, par un enzyme clivant spécifiquement les résidus d'acide sialique, par exemple une neuraminidase de Clostridium perfringens, puis désactiver thermiquement l'enzyme, concentrer la solution et la sécher, par exemple par lyophilisation.

De préférence, on peut cliver les résidus d'acide sialique par hydrolyse par un acide minéral en solution aqueuse diluée, par exemple l'acide chlorhydrique ou sulfurique.

Quelle que soit la méthode de désialylation employée, on purifie de préférence les produits bruts obtenus selon le cas par remise en solution aqueuse ou neutralisation et filtration de la solution sur gel, puis concentration et séchage de la solution purifiée, par exemple par lyophilisation.

Les caséino-glycopeptides peuvent être préparés en faisant subir au caséino-macroglycopeptide, le cas échéant désialylé, une hydrolyse par un enzyme protéolytique.

A cet effet, on peut utiliser n'importe quel enzyme protéolytique actif à pH acide, neutre ou alcalin. L'enzyme peut être d'origine fongique, microbienne (par exemple pronase, alcalase), végétale (par exemple bromeline, ficine, papaïne) ou animale (par exemple trypsine, pepsine, pancréatine). Après le traitement enzymatique, on désactive l'enzyme, par exemple thermiquement, puis on concentre et on sèche la solution, par exemple par lyophilisation, et on purifie de préférence le produit obtenu par remise en solution aqueuse, filtration sur gel puis séchage, par exemple par lyophilisation.

L'agent peut être incorporé dans un article de confiserie, par exemple un bonbon ou une gomme à mâcher. Il peut être dissout par exemple dans une boisson sucrée.

Il peut servir à la désinfection de la cavité buccale et se présenter sous une forme appropriée à cet usage, par exemple en comprimé ou dragée à sucer, en tablette ou comprimé à dissoudre ou par exemple sous forme de solution aqueuse ou émulsion comme bain de bouche.

Il peut être incorporé à une poudre ou pâte dentifrice.

Dans une telle composition, l'agent anti-plaque et anti-carie peut représenter 0,1 à 90% en poids.

Les exemples suivants illustrent l'invention. Dans ceux-ci, les parties et pourcentages sont pondéraux, sauf indication contraire.

Exemples 1 - 6

1. On prépare une solution aqueuse tampon à pH 6,3 de 5 g de caséinate de sodium d'origine bovine dans 100 ml d'eau contenant 10 mM de phosphate de sodium et 100 mM de NaCl. On ajoute à cette solution 1 mg de présure (chymosine Sigma) préalablement dissoute dans la solution tampon. Après incubation à 37°C pendant 1 h, on ajoute lentement et sous agitation une solution à 24% (poids/volume) d'acide trichloracétique (TCA) jusqu'à une concentration finale de 12% de TCA. Il se forme un précipité qu'on sépare par centrifugation et on recueille le surnageant que l'on dialyse contre l'eau pure, puis on sèche le dialysat par lyophilisation. On obtient ainsi 0,8 g de caséino-macroglycopeptide bovin brut.

2. On dissout 320 g d'une poudre de concentré de protéines de petit-lait doux d'origine bovine dans 3,2 l d'eau. Le concentré est obtenu par ultrafiltration puis diafiltration de petit-lait doux et contient en poids des matières sèches 81% de protéines, 1,3% de lactose, 6,3% de graisse et 3,4% de cendres.

A cette solution, on ajoute lentement et sous agitation 3,2 l d'une solution à 24% (poids/volume) de TCA. Il se forme un précipité léger en suspension. Après avoir laissé la suspension obtenue au repos pendant 15-20 min, on la centrifuge à 3000 t/min pendant 15 min. On récupère le surnageant que l'on dialyse contre l'eau pure de manière extensive jusqu'à pH 5, que l'on concentre jusqu'à un volume de 500 ml, puis que l'on dialyse à nouveau contre l'eau pure, que l'on concentre à nouveau et que l'on sèche par lyophilisation. On recueille 7,06 g de caséino-macroglycopeptide bovin brut. Après reprise dans une solution aqueuse 0,1 M d'acide acétique et passage sur une colonne de Sephadex G-25®,

3

éluée avec une solution aqueuse 0,1 M d'acide acétique, on obtient après concentration et lyophilisation 5,52 g de caséino-macroglycopeptide bovin purifié.

3. On acidifie 1 l de lait de chèvre écrémé avec une solution aqueuse d'acide chlorhydrique 1N jusqu'à pH 4,65. La caséine précipite en suspension fine. On laise au repos la suspension à 4°C pendant une nuit. Aprés centrifugation, on recueille le précipité. On le suspend dans 500 ml d'eau et on ajoute 1,7 g de CaCl$_2$ dissout dans 10 ml d'eau sous agitation. On chauffe ensuite le mélange par un bain d'eau à 35°C et on ajoute une solution aqueuse de NaOH 1N jusqu'à pH 6,8. On observe la formation d'une suspension.

Sous agitation lente, on ajoute lentement une solution de 250 µl de présure dans 5 ml d'eau à la suspension, puis après une heure d'agitation à 35°C, on régle le pH à 4,5 avec une solution aqueuse d'HCl. Après centrifugation, on élimine le précipité et on ajoute au surnageant une solution à 24% (poids/volume) de TCA. La suite des opérations s'effectue comme indiqué à l'exemple 2. On recueille 0,25 g de caséino-macroglycopeptide caprin brut. Ce composé peut être purifié par filtration sur gel (Sephadex G 50®) et élué par une solution aqueuse 0,1 M d'acide acétique pour conduire à 0,17 g de caséino-macroglycopeptide caprin purifié.

4. On répète la procédure de l'exemple 3 à partir de 1 l de lait de brebis écrémé et on obtient 0,37 g de caséino-macroglycopeptide ovin brut, respectivement 0,23 g de produit purifié par filtration sur gel.

5. On récolte du petit-lait de chèvre provenant de la fabrication de fromages de chèvre coagulés à la présure immédiatement après fabrication. On mélange 3,5 l de ce petit-lait avec le même volume d'une solution aqueuse à 24% (poids/volume) de TCA. La suite des opérations s'effectue exactement comme décrit à l'exemple 2. On obtient 1,68 g de caséino-macroglycopeptide caprin brut, respectivement 0,66 g de produit purifié après filtration sur gel comme décrit à l'exemple 3.

6. On répète la procédure de l'exemple 5 à partir de petit-lait de brebis et on obtient 2,29 g de caséinomacroglycopeptide ovin brut, respectivement 1,44 g de produit purifié par filtration sur gel comme décrit à l'exemple 3.

Exemples 7-10

7. On dissout 400 mg du caséino-macroglycopeptide brut de l'exemple 1 dans 200 ml d'une solution aqueuse à 50 mM de tampon citrate/phosphate à pH 5. On ajoute à cette solution 40 unités de neuraminidase de Clostridium perfringens (47 mg de type V, Sigma) et on l'incube à 37°C pendant 1 h. Après dénaturation thermique de l'enzyme, on concentre la solution et on la sèche par lyophilisation. Après dissolution du lyophilisat dans 10 ml d'une solution tampon 10 mM d'acide acétique, on filtre la solution sur gel (Sephadex G 50®) avec une solution aqueuse d'acide acétique 10 mM. On obtient un caséino-macroglycopeptide désialylé pur par concentration puis lyophilisation.

8. On procède à l'hydrolyse acide dans des conditions ménagées de 400 mg du caséino-macroglycopeptide brut de l'exemple 2 soit à l'aide de 40 ml d'une solution aqueuse d'HCl 10 mM (pendant 2 h à 80°C), soit avec 40 ml d'une solution aqueuse d'H$_2$SO$_4$ 25 mM (pendant 2 h à 80°C). Après avoir refroidi à la température ambiante, on neutralise la solution à pH 7 à l'aide d'une solution aqueuse de NaOH 0,5 N, puis on concentre et on lyophilise la solution. Après dissolution du lyophilisat dans une solution aqueuse d'acide acétique 0,1 M, on purifie la solution par filtration sur gel Sephadex G 25® avec une solution aqueuse 0,1 M d'acide acétique. Après concentration et lyophilisation, on obtient un caséino-macroglycopeptide désialylé pur.

9. On procède comme à l'exemple 8 à partir du caséino-macroglycopeptide brut de l'exemple 3 et on obtient un caséino-macroglycopeptide désialylé caprin.

10. On procède comme à l'exemple 8 à partir du caséino-macroglycopeptide brut de l'exemple 4 et on obtient un caséino-macroglycopeptide désialylé ovin.

Exemples 11-13

11. On dissout 2 g du produit brut de l'exemple 2 dans 100 ml d'une solution aqueuse de tampon Tris-HCl (Tris (hydroxyméthyl)-aminométhane, 50 mM, amenée à pH 8,2 avec une solution aqueuse d'HCl 1 N). Après addition de 50 mg de Pronase E (Merck), on incube la solution à 40°C pendant 24 h en présence de 5 ml de toluène. On ajoute une nouvelle fois 25 mg de Pronase E et on incube la solution pendant 48 h à 40°C. Après inactivation thermique de l'enzyme, on concentre la solution et on la sèche par lyophilisation. On dissout le lyophilisat dans une solution aqueuse d'acide acétique 0,1 M, puis on filtre la solution sur gel Sephadex G 50® et on élue avec une solution aqueuse d'acide acétique 0,1 M. Après concentration et séchage par lyophilisation, on obtient un caséino-glycopeptide bovin pur.

4

12. On procède comme à l'exemple 11 à la protéolyse du produit brut de l'exemple 8 et on obtient un caséino-glycopeptide bovin désialylé pur.

13. On procède comme à l'exemple 11 à la protéolyse du produit brut de l'exemple 4 et on obtient un caséino-glycopeptide ovin pur.

Exemples 14-15

Souches et conditions de culture

Les souches suivantes ont été utilisées pour prouver l'effet des agents selon l'invention:
- Une souche d'Actinomyces naeslundii ATCC 12104.
- Deux souches d'Actinomyces viscosus (OMZ 105 et OMZ 206), une souche de Streptococcus sanguis (OMZ 9) et une souche de Streptococcus mutans (OMZ 176) qui proviennent du Prof. Dr. B.Guggenheim, "Abteilung für Orale Mikrobiologie und Allgemeine Immunologie, Zahnärztliches Institut, Universität Zürich, Schweiz".

Pour les expériences d'inhibition d'hémagglutination (exemple 14), les souches d'Actinomyces (A. naeslundii ATCC 12104 et A. viscosus OMZ 105) ont été mises en culture dans un milieu du commerce ("Actinomyces broth", BBL, Becton, Dickinson & Co, ou Difco Laboratories). Pour les expériences effectuées sans contrôle de la phase de croissance bactérienne (Tableau I), ce milieu a été complété par l'addition de thioglycolate de sodium (0,05%) et les bactéries ont été récoltées après un passage unique. Pour les expériences effectuées avec une souche récoltée en phase de croissance exponentielle (Tableau II), un premier passage de 24h a été suivi d'un second passage de 16h, les deux fois dans le milieu ci-dessus non complété.

Pour les expériences d'inhibition d'adhésion sur surface de matière plastique (exemple 15), les souches de A.viscosus (OMZ 105 et OMZ 206) ont été cultivées 36h dans le même milieu (non complété). Les souches de Streptococcus (S. sanguis OMZ 9 et S. mutans OMZ 176) ont été mises en culture 24h dans un autre milieu du commerce ("Brain-Heart Infusion", Difco), complété par du glucose (0,5%).

14. Inhibition d'hémagglutination provoquée par des souches d'Actinomyces en présence de kappa-caséino-glycopeptide et dérivés.

Après leur récolte, les bactéries ont été lavées avec de l'eau saline (0,9 % NaCl) et les suspensions ont été ajustées à une densité optique (DO) égale à 8.

Les érythrocytes humains (gr. AR +) ont été désialylés à l'aide de la neuraminidase de Vibrio cholera (Behring), juste avant leur utilisation dans les tests. Après plusieurs lavages, ils ont été suspendus à une concentration de 1% dans de l'eau saline contenant déjà 1% d'$\alpha$-mannoside de méthyle.

Estimation du titre hémagglutinant: Dans une plaque à "Micro-Titre", des portions de 50 $\mu$l des suspensions bactériennes décrites ci-dessus, groupées en séries de dilutions 2 à 2, ont été mélangées à des portions (50$\mu$l) de la suspension d'érythrocytes désialylés également décrite ci-dessus. Les lectures ont été effectuées après une heure d'immobilisation à température ambiante.

Tests d'inhibition d'hémagglutination:Ces tests ont été effectués en mélangeant 25 $\mu$l de la suspension bactérienne correspondant à quatre doses hémagglutinantes (titre déterminé par l'expérience ci-dessus), 50 $\mu$l de la suspension erythrocytaire déjà décrite, et 25 $\mu$l d'une solution saline contenant des séries de dilutions 2 à 2 des différents inhibiteurs. Lorsque cela s'est avéré nécessaire, le pH des suspensions d'inhibiteurs a été amené à 7 avec une solution aqueuse de NaOH 0,5 N, avant d'effectuer le mélange. Les lectures ont été effectuées après une heure d'immobilisation à température ambiante. Les résultats sont indiqués dans les tableaux I et II ci-après:

5

## Tableau I

Inhibition d'hémagglutination d'érythrocytes humains (A$^+$) désialylés

| Produit | | Concentration [a] (mg/ml) | |
|---|---|---|---|
| | | A. viscosus OMZ 105 | A. naeslundii ATCC 12104 |
| Exemple | 2 | 0,63 | 1,25 |
| " | 8 | 0,94 | 1,88 |
| " | 11 | 5 | 10 |
| " | 12 | 2,5 | 5 |
| " | 4 | 4,75 | 9,5 |
| " | 10 | 1,13 | 2,25 |
| " | 13 | 1,88 | 3,75 |
| " | 3 | 1,75 | 3,5 |
| " | 9 | 1,25 | 2,5 |
| Lactose (à titre de comparaison) | | non[b] | non[b] |

Légende:

a) Concentration minimum résultant en une inhibition complète de l'hémagglutination.

b) Pas d'inhibition jusqu'à 20 mg/ml.

## Tableau II

### Inhibitions d'hémagglutination d'érythrocytes humains (A+) désialylés par A.viscosus OMZ (récoltée en phase exponentielle)

| Produit | | Concentration a) (mg/ml) | | Activité relative à l'exemple 12 pris comme référence (x fois) |
|---|---|---|---|---|
| | | exprimée en poids sec / ml | exprimée en équivalent galactoside/ml | |
| Exemple | 2 | 5 | 0,36 | 3 |
| " | 8 | 0,075 | 0,008 | 120 |
| " | 11 | 15 | 2,12 | 0,5 |
| " | 12 | 4,5 | 0,97 | 1 |
| " | 6 | non b) | > 0,34 | - |
| " | 10 | 5 | 0,23 | 4 |
| " | 5 | non b) | > 1,46 | - |
| Lactose (à titre de comparaison) | | non b) | > 10 | - |

### Légende: cf. Tableau I

Il ressort des résultats ci-dessus que les différents agents selon l'invention sont de très forts inhibiteurs des hémagglutinations régies par les pili de type 2. Le Tableau II met particulièrement en évidence la très forte activité du caséino-macroglycopeptide bovin désialylé (selon l'exemple 8). Dans les conditions des essais, l'inhibiteur connu de l'adhésion bactérienne régie par ces pili (le lactose) est ici complétement inefficace.

15. Inhibition de l'adhésion de souches de A. viscosus, S.sanguis et S. mutans sur une surface de matière plastique, en présence de kappa-caséino-glycopeptides bovins et dérivés

Après leur récolte, les bactéries ont été lavées avec une solution aqueuse tampon (0,9 % NaCl, 1mM Tris, 0,1 mM $CaCl_2$, 0,1 mM $MgCl_2$, 0,02 % $NaN_3$), et les suspensions ont été ajustées à une DO égale à 0,4. Dans le cas de A.viscosus OMZ 206, la suspension a dû être réajustée à une DO égale à 0,4, après 6 heures de croissance dans la solution tampon ci-dessus. Des portions de 1 ml de ces suspensions bactériennes ont été transvasées dans des tubes en polypropylène, contenant ou ne contenant pas les différents composés à évaluer. Ces suspensions ont ensuite été agitées pendant 10 s. à chaque heure. Après 4 h et dans chaque cas, la DO de la suspension résultante a été mesurée après transvasement. La différence entre les DO originale et résiduelle a finalement été rapportée à la DO de la suspension de départ (% d'adhésion). Le rapport entre les résultats obtenus en présence et en absence d'inhibiteur a permis de calculer le pourcentage d'inhibition dans chaque cas. Les résultats sont indiqués dans les tableaux III et IV ci-après.

TABLEAU III

Adhésion et inhibition d'adhésion bactérienne sur tubes de plastique

| Produit | S. sanguis OMZ 9 [a] | S. mutans OMZ 176 | A. viscosus OMZ 105 [a] | A. viscosus OMZ 206 |
|---|---|---|---|---|
| | Adhésion (%) | | | |
| Aucun (contrôle) | 90 | 71 | 43 | 37 |
| | Inhibition (%) | | | |
| Exemple 2 [b] (1mg/ml) | 86 | 83 | 93 | 86 |
| Exemple 8 [b] (1mg/ml) | 97 | 80 | 100 | 78 |
| Lactose [b] (1mg/ml) | 12 | 4 | 14 | 15 |
| Galactose [b] (1mg/ml) | 12 | 4 | 12 | n.t. [c] |
| α Mannoside de méthyle (1mg/ml) | 11 | 3 | 10 | n.t. |
| Acide poly-glutamique (1mg/ml) | 6 | 20 | 22 | n.t. |
| Polylysine (1mg/ml) | 12 | 30 | 46 | n.t. |

[a] Dans le cas de ces deux souches, des expériences préliminaires avaient montré la spécificité de l'action inhibitrice des caséino-macroglycopeptides et de leurs dérivés désialylés bovins, par rapport à la non-activité du glucose et de la glycine dans un tel système expérimental.

[b] Avec chacune des quatre souches, d'autres expériences ont montré que cette action inhibitrice des caséino-macroglycopeptides et de leurs dérivés désialylés bovins n'était pas entravée par la présence de concentrations variables (0,05, 0,1, 0,2 et 0,5%) de sucres alimentaires (glucose, saccharose, amidon et fructose).

[c] n.t. = non-testé

8

EP 0 283 675 B1

TABLEAU IV

Inhibition de l'adhésion de S.sanguis OMZ 9 et de S.mutans OMZ 176 sur tubes de plastique à la concentration de 100 $\mu g$ /ml

| Produit | Inhibition % | |
|---|---|---|
| | S. sanguis OMZ 9 | S. mutans OMZ 176 |
| Exemple 2 | 61 | 65 |
| Exemple 8 | 69 | 69 |
| Exemple 11 | 24 | 23 |
| Exemple 12 | 24 | 44 |

Les résultats qui précèdent montrent que les agents selon l'invention sont actifs contre l'adhésion sur une surface de matière plastique qui concerne autant les souches de Streptococci que celle d'Actinomyces. Un tel modèle expérimental est représentatif de la situation qui prévaut dans la cavité buccale.

A titre de comparaison, le lactose et le galactose ne présentent pas du tout les mêmes niveaux d'activité.

**Revendications**

1.  Utilisation des kappa-caséinoglycopeptides ou de leurs dérivés désialylés en tant qu'agents anti-plaque dentaire.

2.  Utilisation des kappa-caséinoglycopeptides en combinaison avec un support adapté à l'administration par voie orale ou topique pour l'obtention d'une composition destinée à traiter la carie dentaire.

3.  Utilisation des dérivés désialylés des kappa-caséinoglycopeptides en tant qu'agents anti-carie dentaire.

4.  Utilisation selon la revendication 1 ou 2 d'un caséino-macroglycopeptides bovin, ovin ou caprin.

5.  Utilisation selon la revendication 1 ou 3 d'un dérivé désialylé d'un kappa-caséinoglycopeptide bovin, ovin ou caprin.

6.  Utilisation selon la revendication 5 d'un dérivé désialylé de caséino-macroglycopeptide bovin.

9

## Claims

1. The use of kappa-caseinoglycopeptides or desialylated derivates thereof as dental anti-plaque agents.

2. The use of kappa-caseinoglycopeptides in combination with a support suitable for oral or topical administration to obtain a composition intended for the treatment of dental caries.

3. The use of desialylated derivatives of kappa-caseinoglycopeptides as dental anti-caries agents.

4. The use claimed in claim 1 or 2 of a bovine, ovine or caprine caseino-macroglycopeptide.

5. The use claimed in claim 1 or 3 of desialylated derivative of a bovine, ovine or caprine caseino-macroglycopeptide.

6. The use claimed in claim 5 of a desialylated derivative of a bovine caseino-macroglycopeptide.

## Patentansprüche

1. Verwendung von Kappa-Kaseinoglykopeptiden oder von deren von Sialinsäuren befreiten privaten als Anti-Zahnbelags-Mittel.

2. Verwendung von Kappa-Kaseinoglykopeptiden in Verbindung mit einem Träger. welcher fur die Verabreichung auf oralem oder topischem Weg vorgesehen ist. um eine zur Behandlung von Zahnkaries bestimmte Zusammensetzung zu erhalten.

3. Verwendnng von Kappa-Kaseinoglykopeptedderivaten, welche von Sialinsäuren befreit sind, als Anti-Zahnkaries-Mittel.

4. Verwendung eines Kaseinomakroglykopeptids aus Rindern, Schafen oder Ziegen nach Anspruch 1 oder 2.

5. Verwendung eines von Sialinsauren befreiten Kappa-Kaseinoglykopeptidderivates aus Rindern, Schafen oder Ziegen nach Anspruch 1 oder 3.

6. Verwendung eines von Sialinsäuren befreiten Kaseinomakroglykopeptidderivates aus Rindern nach Anspruch 5.